# EUROPÄISCHE PATENTSCHRIFT

(11) **EP 0 810 886 B1**
(45) Veröffentlichungstag und Bekanntmachung des Hinweises auf die Patenterteilung: **18.09.2002**
(21) Anmeldenummer: 96904769.5
(22) Anmeldetag: 14.02.1996
(51) Int. Cl.: A61L 15/60, A61L 15/42, C08J 5/18, B32B 5/18

(54) **SCHICHTFÖRMIG AUFGEBAUTER KÖRPER ZUR ABSORPTION VON FLÜSSIGKEITEN SOWIE SEINE HERSTELLUNG UND VERWENDUNG**
LAYERED BODY FOR ABSORBING LIQUIDS, ITS PRODUCTION AND USE
CORPS STRATIFIE POUR L'ABSORPTION DE LIQUIDES, SA PRODUCTION ET SON UTILISATION

(30) Priorität: 20.02.1995 DE 19505709
(43) Veröffentlichungstag der Anmeldung: 10.12.1997
(73) Patentinhaber: Chemische Fabrik Stockhausen GmbH, 47805 Krefeld (DE)
(72) Erfinder: BRÜGGEMANN, Helmut, D-47057 Duisburg (DE); DAHMEN, Kurt, D-41239 Mönchengladbach (DE); LEHWALD, Dieter, D-50829 Köln (DE); THEILMANN, Roland, D-47805 Krefeld (DE)
(74) Vertreter: Kahlhöfer, Hermann, Dipl.-Phys.
(86) Internationale Anmeldenummer: EP9600620
(87) Internationale Veröffentlichungsnummer: WO96025958

(56) Entgegenhaltungen:
- EP-A- 0 378 940
- WO-A-87/00438
- WO-A-94/25519
- FR-A- 2 203 827
- US-A- 4 000 028
- US-A- 4 335 722

## Beschreibung

Die Erfindung betrifft Wasser und wäßrige Flüssigkeiten absorbierende Körper, die aus geschäumten, löslichen Polymerschichten und superabsorbierenden Polymeren bestehen, sowie ein Herstellungsverfahren für diese Körper und deren Verwendung als Absorptionsmittel, insbesondere im Hygienebereich zur Aufnahme von Körperflüssigkeiten, wie Blut, Schweiß, Urin und anderen flüssigen Ausscheidungen. Weiterhin betrifft die Erfindung die Verwendung der Körper als Komponenten in Wundabdeckungen, in Verpackungs- und Isolationsmitteln, in Textilien für Bekleidungs- und Reinigungszwecke, sowie die Verwendung im Bereich der Pflanzenzucht und als Depotmaterial.

Die Verwendung von superabsorbierenden Polymeren geschieht heute vorwiegend in pulverförmiger Ausführung. Im Zuge von Verfahrensvereinfachungen besteht der Wunsch, die Superabsorber in einer fixierten Form einzusetzen, d.h. in in einer Matrix einzubinden. Gemäß dem Stand der Technik gibt es eine Reihe von Lösungsvorschlägen, die jedoch alle mit Mängeln behaftet sind.

Schichtförmig aufgebaute Körper mit der Fähigkeit wäßrige Flüssigkeiten aufzunehmen sind bekannt. Die US 4 000 028 beschreibt Körper aus Latexschaum und Cellulose-Fluff, die jedoch keine superabsorbierenden Polymerisate enthalten, so daß sie eine sehr begrenzte Aufnahmekapazität für Flüssigkeiten besitzen.

In der US 5 128 082 werden absorbierende Körper beschrieben, die aus Mischungen von Fluff-Materialien und superabsorbierenden Polymerisaten sowie einem umgebenden, jeweils die äußere Schicht bildenden Latex hergestellt werden. Der Polymerisatanteil in diesen Körpern ist ungleichmäßig verteilt, was zu den bekannten Schwierigkeiten bei der Aufnahme von Flüssigkeiten und den damit verbundenen Nachteilen in Bezug auf den Tragekomfort dieser Hygieneartikel führt.

In der EP 212 618 B1 werden Windelkonstruktionen beschrieben bei denen zur Vermeidung solcher Nachteile die Polymerisate mit bestimmter Korngrößenverteilung unter Anwendung eines Gradienten in einer Cellulosefaserschicht verteilt werden. Solche Konstruktionen sind jedoch nicht ausreichend stabil, insbesondere ändert sich die Verteilung der Materialien beim Transport.

Das Mischen superabsorbierender Polymerisate mit wasserhaltigen Polymerschäumen führt normalerweise dazu, daß die Schäume unter Wasserentzug zusammenbrechen, wobei die offenzellige Struktur zerstört wird, so daß nachfolgend nur die an der Oberfläche befindlichen SAP - Partikel in der Lage sind, Flüssigkeiten sofort aufzunehmen. Die übrigen SAP - Partikel können erst mit Verzögerung nach dem Auflösen der umgebenden wasserlöslichen Polymerschicht Wasser binden.

Nach EP 427 219 A2 sind Mischungen aus superabsorbierenden Polymeren und Latexschäumen bekannt, die dadurch erhalten werden, daß die Polymerisate als Pulverspray in den aufgeschäumten Latex eingebracht werden. Die Verfahrensweise gestattet keinen definierten Aufbau solcher Körper, insbesondere ist eine genaue Verteilung der Polymerisate nicht möglich.

Aus der EP 577 233 A1 ist die Verwendung eines Bandes als Bestandteil der Isolation von Stromkabeln bekannt, das aus einer Vliesstoffschicht und einer Schaumstoffschicht besteht und Teilchen eines Quellpulvers, die im Bereich der Vliesstoffschicht undefiniert verankert sind, enthält.

Das Patent US 4 649 164 beschreibt geschäumte wasserabsorbierende Materialien, die aus CO₂- freisetzenden Blähmitteln und Acrylat-(Meth)acrylsäure-Latices gebildet werden, wobei der geschäumte Latex selbst das Absorbermaterial darstellt. Aufgrund des hydrophoben Charakters der Acrylatkomponente ist die Aufnahmefähigkeit dieser Schäume gegenüber den bekannten Superabsorbern begrenzt.

Ebenso sind aus der DE 42 42 015 A1 bioverträgliche, als Wundverband verwendbare, offenporige Polyurethanschäume mit Guar gum als eingelagertes Hydrogel bekannt, wobei die Gelkomponente bei der Herstellung in situ eingeschäumt wird. Die Wasseraufnahmekapazität dieser Produkte soll auf einen Wert unterhalb des dreifachen des Ausgangsgewichts begrenzt bleiben.

In der EP 340 945 A1 werden Mischungen von Elastomeren und kationischen, wasserquellbaren Hydrocolloiden, vorzugsweise Chitosansalze, zur Verwendung als Wundabdeckung mit Absorptionswerten von wenigstens 180 Gew.% beschrieben, wobei die Colloidpartikel regellos im Elastomeren eingebunden sind und die Aufnahmefähigkeit für wäßrige Flüssigkeiten ebenfalls gering ist.

In ähnlicher Weise sind aus der DE 42 33 289 A1 hydrophile Polyurethanschaumgele bekannt, die aus Mischungen von Polyolen, Diisocyanaten und superabsorbierenden Polymerisaten hergestellt werden, wobei das superabsorbierende Polymerisat durch das herstellungsbedingte Mischen der Komponenten gleichmäßig im Schaum eingebunden ist. Die Produkte werden als Wundauflagen mit definiertem Haftverhalten eingesetzt.

Die EP 547 474 A1 beschreibt ein Verfahren zur Herstellung von absorbiernden Materialien, in denen superabsorberierende Polymere verteilt sind. Die so erhaltenen absorbierenden Materialien besitzen eine Absorptionskapazität, die geringer ist, als es dem Anteil des in den Materialien eingearbeiteten SAP entsprechen würde, d.h., daß aufgrund der Auswahl der verwendeten Materialien und des angewendeten Herstellungsprozesses ein Teil des SAP blockiert wird. Die Art des verwendeten Matrixmaterial ist auch insofern eingeschränkt, daß der Schmelzpunkt dieses Materials über der Zersetzungstemperatur des SAP liegen muß.

Die EP 303 445 A1 beschreibt ein absorbierendes Flächengebilde, in dem auf einem Träger ein wasserhaltiges SAP fixiert wurde. Die Verwendung diese Gebildes ist auf ein Pflaster als Medikamentenreservoir eingeschränkt.

Die JP Appl No. 75 - 85462 beschreibt eine Methode zur Herstellung von superabsorbierenden Flächen aus einem wasserabsorbierenden Material, das aus einem Stärke-Pfropfpolymeren besteht und in einem wasserlöslichen, filmbildenden Polymeren eingebunden ist. Als unverzichtbarer Bestandteil wird in dieser Erfindung als dritter Bestandteil ein Material genannt, das als Basismaterial fungiert. Das superabsorbiernde Polymere wird mit dem löslichen, filmbildenden Polymeren auf diesem Basismaterial fixiert.

EP 604 730 A1 beschreibt SAP enthaltende Gebilde, die in Wasser zerfallen. Neben dem SAP sind dispergierbare Polymere und Weichmacher als unverzichtbare Komponenten genannt. Die Gebilde erfüllen nicht die Forderung nach einer definierten Anordnung eines Superabsorbers in einer Matrix, da die in dieser Schrift beschriebenen Verfahren wie Extrudieren, Mischen oder Vermengen dazu völlig ungeeignet sind. Nach dem Desintegrieren der beschriebenen Flächengebilde verbleiben neben dem Superabsorber auch Partikel; das beschriebene Matrixmaterial ist also nicht wasserlöslich.

Es bestand daher die Aufgabe, einen Körper auf der Basis von schichtförmig konstruierten Absorptionsmaterialien für Wasser und wäßrige Flüssigkeiten bereitzustellen, der die geschilderten Nachteile vermeidet, d.h. der insbesondere eine durch die Struktur unbehinderte Aufnahme von Wasser und wäßrigen Flüssigkeiten durch den Superabsorber ermöglicht, der eine rasche Aufnahmegeschwindigkeit zeigt, der eine definierte Anordnung des superabsorbierenden Polymers im Körper ermöglicht, der technisch einfach herzustellen ist und der aufgrund seiner mechanischen Stabilität und Flexibilität vielseitig einsetzbar ist.

Die Aufgabe konnte durch einen schichtförmigen Körpers aus mindestens einer wasserlöslichen Polymerschaumschicht und mindestens einer aus partikelförmigem, superabsorbierenden Polymerisat gebildeten Schicht gelöst werden, wobei der schichtförmige Körper die Menge des superabsorbierenden Polymerisats in bestimmter Verteilung und fixiert an der Grenzfläche der Schaumschicht enthält.
In Konstruktionen mit mehreren Schichten an Schaum und superabsorbierenden Polymerisat können auch solche Körper erhalten werden, die sowohl eine definierte Verteilung des Superabsorbers in jeder einzelnen Fläche als auch eine definierte Verteilung des superabsorbierenden Polymeren (z.B. einen Gradienten) quer zu den einzelnen Schichten aufweisen.

Gegenstand der Erfindung ist demnach ein schichtförmiger aus einer oder mehreren wasserlöslichen Polymerschaumschichten und partikelförmigen superabsorbierenden Polymerisaten bestehender Körper zur Absorption von Wasser und wäßrigen Flüssigkeiten, der dadurch gekennzeichnet ist, daß direkt auf, zwischen oder unter den geschäumten, wasserlöslichen Polymerschichten das superabsorbierende Polymerisat in mengenmäßig und/oder örtlich vorgegebener und fixierter flächenmäßiger Anordnung enthalten ist und das Mengenverhältnis von geschäumtem, wasserlöslichen Polymeren zum superabsorbierenden Polymerisat 1 : 500 bis 50 : 1, vorzugsweise 1 : 50 bis 25 : 1 und besonders bevorzugt 1 : 25 bis 10 : 1 beträgt. Der schichtförmige Absorberkörper kann starr oder flexibel sein.

Überraschenderweise bleibt beim Absorptionsvorgang unter Verwendung des erfindungsgemäßen schichtförmigen Körpers trotz des direkten Kontakts zwischen geschäumten, wasserlöslichen Polymeren und dem superabsorbierenden Polymeren das Quellvermögen des superabsorbierenden Polymerisats unbeeinträchtigt, während sich die Quellgeschwindigkeit des superabsorbierenden Polymeren sowohl über die Art als auch über den Grad der Schäumung des Matrixmaterials bestimmen läßt.

Der erfindungsgemäße schichtförmige Absorberkörper weist bevorzugt bei Verwendung einer 0,9%igen NaCl-Lösung eine Retention von mindestens 0,1 Liter/m² Oberfläche, eine Aufnahme von mindestens 0,1 Liter/m² Oberfläche und eine Absorption unter Belastung (AUL) von mindestens 2 g/g bei 0,021 Pa auf.

Gegenstand der Erfindung ist weiter ein Herstellungsverfahren sowie die Verwendung der erfindungsgemäßen schichtförmigen Absorberkörper. Das Herstellungsverfahren ist dadurch gekennzeichnet, daß wenigstens
a) der Schaum eines wasserlöslichen Polymers mit einem Litergewicht von 10 bis 1000 g/l erzeugt und
   der Schaum flächenförmig in einer Schichtdicke von 1 µm bis 100.000 µm, vorzugsweise von 10 µm bis 10.000 um und besonders bevorzugt von 200 µm bis 5.000 µm verteilt wird,
b) das superabsorbierende, partikelförmige Polymerisat in einem Mengenverhältnis von geschäumter, wasserlöslicher Polymerschicht zum superabsorbierenden Polymerisat von 1 : 500 bis 50 : 1, vorzugsweise 1 : 50 bis 25 : 1 und besonders bevorzugt 1 : 5 bis 10 : 1 gegebenenfalls unter Verwendung mindestens einer Schablone, einer Lochscheibe und/oder eines Siebes in bestimmter mengen- und flächenmäßiger Verteilung auf den flächenförmig verteilten Schaum aufgebracht, gegebenenfalls durch Wärmebehandlung fixiert wird, wobei gegebenenfalls der Verfahrensschritt a) und/oder b) in beliebiger Reihenfolge wiederholt wird und abschließend eine Wärmebehandlung, ggf. unter schwacher Vernetzung der geschäumten Schichten erfolgt.

Als Grundlage für die geschäumte, wasserlösliche Polymerschicht kommen sowohl synthetische, wasserlösliche Polymere wie z.B. Polyvinylalkohole, Polyalkylallylether, Polyglykolether, Polyvinylpyrolidone, Polyacrylate , Polymethacrylate sowie Derivate und Copolymere davon als auch natürliche, wasserlösliche Polymeren wie z.B. Guar, Alginate, Agar Agar, Xanthan, Pektin, Stärke u.ä. sowie chemisch modifizierte Rohstoffe wie z.B. Ether und/oder Ester und/oder Hydrolysate und/oder Oxidationsprodukte von Polysacchariden oder Proteinen wie z.B. Cellulose, Amylose, Stärke oder Weizenkleber in Frage sowie Copolymerisate und/oder Pfropfpolymerisate auf Basis natürlicher oder synthetischer Polymere.

Die Auswahl des Matrixmaterials hängt nicht zuletzt vom beabsichtigten Einsatzzweck ab. Aufgrund der optional möglichen Kombination von natürlichen Polymeren sowohl in der Matrixkomponente als auch in der SAP-Komponente bietet sich damit ein Weg zur Herstellung von biologisch leicht abbaubaren absorbierenden Körpern an.
Durch das Matrixmaterial läßt sich die Flexibilität der superabsorbierenden Fläche in weiten Breiten variieren. Bei vorgegebener Matrix kann die Flexibilität und Festigkeit der superabsorbierenden Fläche auch durch Zuschlagstoffe wie 2-Ethylhexanol, Glycerin, Phthalsäureester u.ä., jedoch auch durch Füllstoffe wie Kreide, Pigmente, Fasern u.ä. verändert werden.

Das Schäumen des wasserlöslichen Polymers wird mit bekannten Mitteln, beispielsweise durch starkes Rühren oder Mixen einer Polymerlösung unter Vermischung mit Luft in der Regel unter Zusatz von Schaumhilfsmitteln erreicht. Optional können dem zu verschäumenden Material auch Treibmittel, wie beispielsweise Ammoniumcarbonat oder Azodicarbonamid zugesetzt werden. Hierdurch entsteht die Möglichkeit, daß die wasserlöslichen Polymeren nicht als Lösung, sondern in Masse aufgeschäumt werden. Beispielsweise kann eine Polymerschaummatrix durch Extrusion erzeugt und im noch plastischen Zustand mit dem superabsorbierenden Polymer bestreut werden.
Es ist darüber hinaus auch möglich, die Schaummatrix aus dem wasserlöslichen Polymer nach dem Extrudieren und Erstarren oberflächlich durch Befeuchtung geringfügig klebrig zu machen und dann das superabsorbierende Polymer darauf zu fixieren.
Die Art und Menge des eingesetzten Matrixmaterials bestimmt in bekannter Weise die mechanischen Eigenschaften der erfindungsgemäßen Körper, wie z.B. den Grad der Flexibilität sowie das Oberflächenverhalten der Konstruktionen.

Es wurde festgestellt, daß sich die Aufnahmegeschwindigkeit von Wasser oder wäßrigen Flüssigkeiten wesentlich von der Art und dem Grad der Schäumung des verwendeten Polymerschaums bestimmt wird. Superabsorbierende Flächengebilde mit schwach oder nicht geschäumte Schichten des löslichen Polymers weisen im Vergleich zu solchen mit hoch aufgeschäumten Schichten des löslichen Polymers geringere Aufnahmegeschwindigkeit für Wasser bzw. wässrige Lösungen auf.

Außerdem findet eine starke Beeinflussung der mechanischen Stabilität durch die Art und Menge sowie die Verteilung von zusätzlich eingesetzten Füllstoffmaterialien statt.

Geeignete Füllmaterialien sind Kreiden, Bentonite, Kieselgele und Kieselsäure, Aktivkohlen, Pigmente, wie Titandioxid und Eisenoxid, sowie natürliche und/oder synthetische Fasermaterialien, wie beispielsweise Viskose- und Baumwollfasern und -gewebe und/oder Polyester- und Polyamidfasern und Mischungen verschiedener Fasern oder entsprechender Gewebe. Weiterhin sind feingemahlene Kunststoffe geeignet. Die Art, Konzentration und Verteilung des Füllmaterial kann in jeder Schaumschicht gleich oder verschieden sein. Ebenso können Mischungen verschiedener Füllstoffe verwendet werden. Die einzelne Schaumschicht kann einen Füllungsgrad von 0 bis 1000 Gew.%, bezogen auf die Menge des wasserlöslichen Polymeren, bevorzugt maximal 400 Gew.% und besonders bevorzugt maximal 200 Gew.% aufweisen. Darüber hinaus können die beschriebenen Füllmaterialien auch als separate Schicht in den absorbierenden Körper eingebracht werden. Das superabsorbierende Polymerisat kann auch als Mischung mit den als Füllstoff genannten Materialien aufgebracht werden.

Der Polymerschaum kann in geometrisch verschiedenen Formen gebildet werden, wobei die Erzeugung einer flächenförmigen Schaumschicht (mit beliebiger Dicke) bevorzugt ist. Hierbei können, wie in US 4 000 028 angegeben, zur Herstellung abtrennbare Flächen von Hilfsträgern, wie Metallbänder und Folien, Siliconpapier, Glasfasern, Glasflächen oder textile Gewebe, hilfsweise verwendet werden oder es können erfindungsgemäß vorzugsweise Flächen von Materialien, wie flüssigkeitsdurchlässige und -undurchlässige Kunststoffolien und Vliese, Zellstoff- oder Papierschichten oder textile Gewebe als Basis eingesetzt werden, die Bestandteil des absorbierenden Körpers werden.

Die partikelförmigen, superabsorbierenden Polymerisate können erfindungsgemäß aus wasserunlöslichen, wasserquellbaren Polymerisaten oder Copolymerisaten aus Monomereneinheiten von (Meth)acrylsäure, Maleinsäure, Itaconsäure sowie deren Anhydriden und Salzen, Fumarsäure und deren Salzen, insbesondere deren Alkali-, Erdalkali und Amoniumsalzen, (Meth)acrylamid, (Meth)acrylnitril und Vinylacetat und dessen Hydrolyseprodukten, Vinylpyrrolidon, Vinylpyrridin, Vinylsulfonsäure und deren Estern und Amiden sowie von N-Alkyl- und NN-Dialkyl- substituierten Estern und/oder Amiden der (Meth)acrylsäure und deren Salzen und/oder deren quartären Amoniumverbindungen bestehen. Ebenso sind natürliche wasserquellbare Polymerisate, wie Carboxymethylcellulose, Hydroxyethylcellulose, Hydroxypropylcellulose, Methylcellulose, Guarkernmehl, Xanthane, Alginate, Stärke und deren.Derivate sowie Pfropf-Polymerisate aus diesen Stoffen und den genannten Monomeren oder Mischungen der zuvor genannten Polymerisate mit diesen Stoffen verwendbar.

Das partikelförmige, superabsorbierende Polymerisat wird auf die zuvor hergestellte Oberfläche der wasserlöslichen Polymerschaumschicht in verteilter Form als Pulver mit einer Kornverteilung von 1 µm bis 20.000 µm aufgebracht. Dies kann beispielsweise durch Aufstreuen des Pulvers aus geeigneten Behältern oder mittels geeigneter Vorrichtungen erfolgen.

Die Korngröße der Pulver ist von der Verwendung der absorbierenden Körper abhängig. Im Hygienebereich werden Pulver mit Korngrößen zwischen 50 µm und 1.000 µm bevorzugt, während bei der Verwendung zur Kabelisolierung ein Bereich unter 400 µm gewählt wird.

In den erfindungsgemäßen Körpern werden auch die Feinstkornanteile der superabsorbierenden Polymerpulver fixiert, so daß Staubprobleme bei der späteren Anwendung bzw. Verarbeitung ausgeschlossen sind. Für gewöhnlich verursachen diese Feinstkornanteile bei der konventionellen Pulververarbeitung/Anwendung außer der Staubentwicklung auch das sogenannte "Blocken" während der Flüssigkeitsaufnahme, bei dem sich die Feinstanteile unter Feuchtigkeitseinfluß zu einer, den Flüssigkeitstransport behindernden Schicht agglomerieren. Diese Probleme werden durch die erfindungsgemäßen Schaumkörper vermieden.

In einer speziellen Ausführungsform kann die Menge und die Verteilung des Pulvers bezogen auf die Flächeneinheit so erfolgen, daß nur bestimmte Oberflächenbereiche der Schaumschicht belegt und/oder die Flächen mit unterschiedlichen Mengen belegt werden. Hierbei kann die Auftragung unter Verwendung von Schablonen, Lochplatten, Sieben oder geeigneten Kombinationen daraus, gegebenenfalls unter Klassierung der Partikelgröße, der Polymerisate erfolgen. Beispielsweise kann durch die Auftragung von Pulvern in feinkörniger Form eine flüssigkeitsblockierende Schicht oder durch Auftragen grobkörniger Polymerisatanteile entgegengesetzt eine verbesserte Verteilung der Flüssigkeit erreicht werden.

Die Menge, Korngröße und Verteilung des partikelförmigen, superabsorbierenden Polymerisats auf den einzelnen geschäumten, wasserlöslichen Polymerschichten kann verschieden sein.

Die Flächenbelegung der Polymerschaumfläche liegt im Bereich von 0,1 g bis 500 g des partikelförmigen, superabsorbierden Polymerisats, bezogen auf einen m² der geschäumten Oberfläche des Körpers, vorzugsweise von 10 bis 300 g/m² und besonders bevorzugt von 50 bis 200 g/m².

Der Anteil des superabsorbierenden Polymerisats an der Gesamtkonstruktion des absorbierenden Körpers beträgt 5 - 99 Gew.%, vorzugsweise 40 - 97,5 Gew.% und besonders bevorzugt 50 - 95 Gew.%.

Die Herstellung des absorbierenden Körpers erfolgt durch Auftragen einer oder mehrerer wasserlöslicher Polymerschaumschichten im Wechsel mit dem Auftrag des partikelförmigen Superabsorbers auf die bereits erzeugte Schicht. Bei unterschiedlichem Aufbringen der Polymerisatpartikel auf bzw. in einzelnen Schichten werden insgesamt Körper hergestellt, in denen das absorbierende Polymerisat mit einem bestimmten Gradienten verteilt ist. Abschließend erfolgt eine Trocknung zur Stabilisierung der wasserlöslichen Polymerschaumschichten bei Temperaturen zwischen den üblicherweise beim Gefriertrocknen angewandten Temperaturen und 300°C, vorzugsweise bei Temperaturen zwischen 50°C und 240°C, gegebenenfalls unter vermindertem Druck, getrocknet. Zum Trocknen des Flächengebildes können auch die Mikrowellentechnik oder Gefriertrocknungstechniken angewendet werden.
Im Zuge der Herstellung der schichtförmige Körper, insbesondere bei deren Trocknung, kann es gegebenenfalls zu chemischen bzw. physikalischen Bindungen zwischen dem Matrixmaterial B und der absorbierenden Komponente A kommen. Als Beispiel für eine chemische Bindung sei hier die Veresterungsreaktion genannt, die zwischen Carboxylund Hydroxylgruppen stattfinden kann. Physikalische Bindungen ergeben sich z.B. durch Verschlaufungen oder Verhakungen der Polymermoleküle im Oberflächenbereich der Komponente A bzw. durch Wechselwirkungen funktioneller Gruppen der Polymermoleküle in den Komponenten A und B.

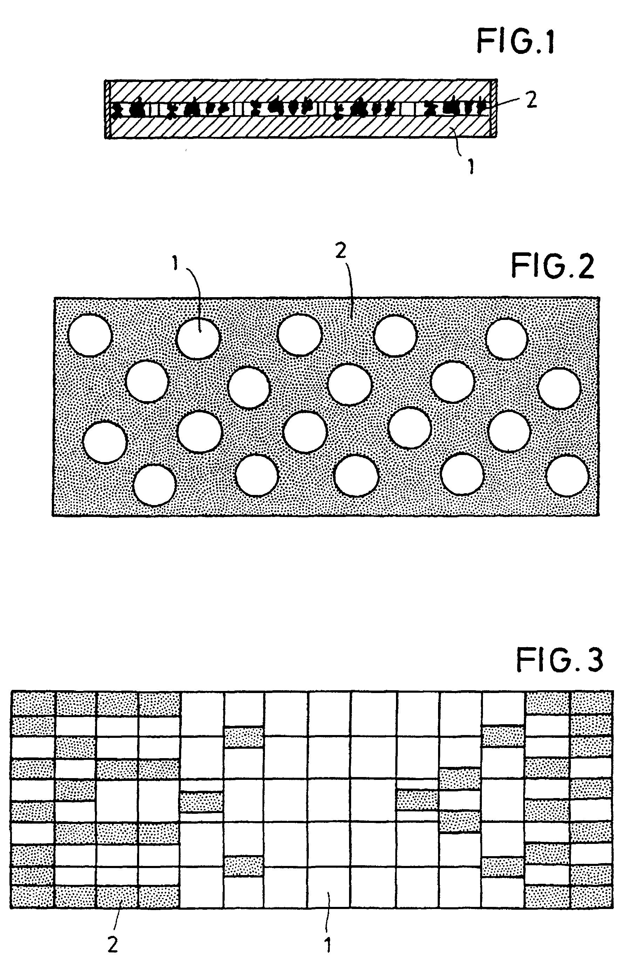

Der erfindungsgemäße Körper kann gegebenenfalls abschließend mit einem Kalander und/oder mit einer Prägewalze bearbeitet werden.
Ein bevorzugtes Beispiel des erfindungsgemäßen absorbierenden Körpers zeigt die Figur 1.

Die erfindungsgemäßen Körper sind zur Absorption von Wasser und wäßrigen Flüssigkeiten verschiedenster Art verwendbar. Sie finden insbesondere direkt oder als Komponente oder als Zusatz von Artikeln für den Hygiene- und Pflegebereich in Windeln, Tampons und in Inkontinenzartikeln sowie in Sanitärartikeln zur Wundabdeckung Verwendung. Weiterhin sind die absorbierenden Körper als Wasser und wäßrige Lösungen speicherndes Pflanzenwuchsmedium, zur Lagerung und zum Transport von Pflanzen und Pflanzenteilen, zur Isolation von Rohren und Leitungen, insbesondere für elektrische und lichtleitende Kabel und als Bestandteil von Bauteilen, beispielsweise zur Isolation von Außenmauern und als Verpackungsmittel oder -komponente für Handelswaren, insbesondere für Lebensmittel und Getränke geeignet. Weiterhin können sie zur Verbesserung des Tragekomforts in Bekleidungsstücke eingearbeitet werden.

Die Eigenschaften der erfindungsgemäßen Wasser und wäßrige Flüssigkeiten absorbierenden Körper können durch die im folgenden dargestellten Testmethoden erfaßt werden.

### Testmethoden:

### Teebeuteltest (TBT)

Zur Bestimmung des Absorptionsvermögens wurde der TBT durchgeführt. Als Prüflösung wurde (soweit nicht anders erwähnt) eine 0,9%ige NaCl-Lösung verwendet.

Aus der absorbierenden Fläche wird ein Stück Material ausgestanzt, das etwa 0,2 g des SAPs enthält. Dieses Stück wird in einem Teebeutel eingewogen. Anschließend wird der Teebeutel für eine definierte Zeit in die Testlösung gelegt. Nach fünfminütiger Abtropfzeit wurde der Teebeutel ausgewogen (Bestimmung des TBT max.), anschließend wurde der Teebeutel in einer Zentrifuge (handelsübliche Wäscheschleuder, 1400 Upm) abgeschleudert. Danach wurde wiederum ausgewogen (Bestimmung des TBT ret. (Retention)).
Durch mehrere Tests mit dem selben Material und unterschiedlichen Tauchzeiten kann die Aufnahme als Funktion der Tauchzeit (Aufnahmegeschwindigkeit) des superabsorbiereneden Flächengebildes für Wasser bzw. wässrige Lösungen bestimmt werden. Die Flüssigkeitsaufnahme wird entweder auf 1 g der Fläche, auf 1 g des eingesetzten SAPs oder auf 1 m² der Fläche berechnet.

### Absorption under Load (AUL)

Um das Flüssigkeitsaufnahmevermögen unter Druck zu bestimmen, wurde die "Absorption under Load", wie in der EP-A-0 339 461 beschrieben, bestimmt.

Abweichend von dieser Vorschrift wurde ein kreisrundes Stück des superabsorbierenden Körpers von der Größe des Innendurchmessers des AUL-Tiegels als Prüfsubstanz eingesetzt. Die Flüssigkeitsaufnahme wurde entweder auf 1 g des Körpers, auf 1 g des eingesetzten SAPs oder auf 1 m² des Körpers berechnet.

### Figurenbeschreibung:

### Figur 1

Ansicht eines erfindungsgemäßen Körpers
1 Schaumschicht
2 Superabsorbierendes Polymer

### Figuren 2 - 3

Streurahmen
1 (helles Feld) durchlässiger Teil des Streurahmens
2 (dunkles Feld) undurchlässiger Teil des Streurahmens

### Figur 4

Windelkonstruktion
1 Laminate aus Polypropylen-Abdeckvlies und Polyethylen-Folie
2 Auslaufschutz mit eingearbeiteten Gummifäden
3 Abdeckvlies aus Polypropylen
4 Polyethylenfolie auf der Rückseite
5 Umhüllung des Kerns aus Cellulosefasern
6 Kern, der den superabsorbierenden Körper enthält

Die Erfindung wird an den nachstehenden Beispielen erläutert.

### Beispiele 1-4

Aus wasserlöslichem Polyvinylalkohol (6 g) und 40 g deionisiertem Wasser wird eine Lösung bereitet. Der Lösung werden 1 g Stokal SR (35 %ige Succinamat - Paste) und 2 g Alkylpolyglykosid zugesetzt. Mit einem Handmixgerät wird die Lösung auf ein Schaumlitergewicht von ca. 50 g/l aufgeschlagen. Ein Teil dieses Schaums wird auf einer Fläche (teflonisierte Folie o.ä.) von 400 cm² gleichmäßig ausgestrichen. Die so entstandene Fläche wird mit 12,4 g Superabsorber FAVOR®SXM 100 (schwachvernetztes, teilneutralisiertes Polyacrylat) bestreut (hierzu wird auf die Fläche eine Schablone - vgl. Fig.2 - aufgesetzt) und anschließend mit dem Rest des Schaums abgedeckt. Dann wird 20 Minuten bei einer Temperatur von 140°C getrocknet. Es werden flexible, superabsorbierende Flächen erhalten, die sich leicht von der Oberfläche (teflonisierte Folie) ablösen lassen.

**Tabelle 1:**

| Die Tabelle zeigt die Abhängigkeit der Aufnahmegeschwindigkeit von der Art des verwendeten Polyvinylalkohols. | | | | |
|---|---|---|---|---|
| Bsp. | PVA - Typ | TBT (1 min.) | IBT (5 min.) | TBT (30 min.)* |
| | | max./ret. | max./ret. | max./ret. |
| | | [g/g]/[g/g] | [g/g]/[g/g] | [g/g]/[g/g] |
| 1 | Mowiol 4/88 | 13/13 | 25/20 | 50/31 |
| 2 | Mowiol 5/88 | 12/12 | 27/21 | 50/31 |
| 3 | Vinex 2144 | 16/15 | 29/24 | 50/31 |
| 4 | Vinol 205 | 15/15 | 27/22 | 50/31 |
| SXM 100 | 0 | | | 50/31 |

| | | | | |
|---|---|---|---|---|
| (die mit * gekennzeichneten TBT Werte beziehen sich auf die Menge an eingesetztem Superabsorber, die anderen TBT Werte beziehen sich auf das Flächengewicht.) | | | | |

### Vergleichsbeispiele 1-4

Es wird wie in den Beispielen 1-4 verfahren, jedoch wird auf das Verschäumen der Komponenten verzichtet. Nach dem Trocknen entsteht somit ein PVA - Film in dem der Superabsorber eingebunden ist.

**Tabelle 2:**

| Die Tabelle zeigt die deutliche Reduzierung der Aufnahmegeschwindigkeit gegenüber den Beispielen 1-4. | | | | |
|---|---|---|---|---|
| Vergl.-Bsp. | PVA - Typ | TBT (1 min.) | TBT (5 min.) | TBT (30 min.)* |
| | | max./ret. | max./ret. | max./ret. |
| | | [g/g]/[g/g] | [g/g]/[g/g] | [g/g]/[g/g] |
| 1 | Mowiol 4/88 | 7/7 | 17/14 | 50/31 |
| 2 | Mowiol 5/88 | 6/6 | 21/17 | 50/31 |
| 3 | Vinex 2144 | 9/9 | 22/18 | 50/31 |
| 4 | Vinol 205 | 5/5 | 15/15 | 50/31 |
| SXM 100 | 0 | | | 50/31 |

### Beispiel 5

Aus 1,7 g Methylan® (handelsüblicher Tapetenkleister auf Basis von Methylcellulose), 2 g Alkylpolyglykosid, 2 g Stokal® SR, 2 g Polydiol 400 und 110 g Wasser wird eine Lösung bereitet. Mit einem Handmixgerät wird daraus ein Schaum mit einem Schaumlitergewicht von ca. 50 g/l bereitet. Die Hälfte des Schaums wird auf einer Fläche von 10 x 40 cm ausgestrichen. Dann wird mit 12,4 g Favor® SXM 100 bestreut (hierzu wird auf die Fläche eine Schablone - vgl. Fig. 2 - aufgesetzt) und anschließend mit der 2ten Hälfte des Schaums abgedeckt. Das Flächengebilde wird 20 Minuten bei 140°C getrocknet.
Es entsteht ein flexibles Flächengebilde mit folgenden Aufnahmecharakteristika: TBT: max./ret. [l/m²]/[l/m²] = 15,4/9,6; AUL (2∗10³Pa) = 9,5 l/m²

### Beispiel 6 - 8

Es werden aus 2 g Tylose (Carboxymethylcellulose), 2 g Alkylpolyglykosid, 2 g Stokal SR, 90 g Wasser, 12,4 g Favor SXM und mit Zusatz einer Weichmacherkomponente auf der in Beispiel 5 beschriebenen Weise absorbierende Flächen hergestellt. Aufnahmecharakteristik der Beispiele 6 - 8: TBT: max./ret. [l/m2]/[l/m2] = 15,4/9,6 AUL (2*103Pa) = 9,5 l/m2

**Tabelle 3:**

| Die Tabelle zeigt die Abhängigkeit der Biegbarkeit der Flächegebilde in Abhängigkeit von der Art und der Menge des eingesetzten Weichmachers | | |
|---|---|---|
| Bsp. Nr. | Weichmacher | Bewertung |
| | | |
| 6 | 4,3 g Glycerin | weich, biegsam, nicht reißfest |
| 7 | 2,0 g Glycerin | mäßig biegsam, brüchig |
| 8 | 2,0 g Edenol B35¹⁾ | kaum biegsam, brüchig |

| | | |
|---|---|---|
| ¹⁾ Fettepoxidat der Firma Henkel | | |

### Beispiel 9 (a/b)

Aus 0,2 g Guarkernmehl, 50 g Wasser, 1 g Stokal SR, **(a)**:3 g bzw. **(b)**: 0 g Cellulosefasern (Nadelholzfasern zur Papierherstellung) und 1,5 g Alkylpolyglykosid wird wie in den vorangegangenen Beispielen beschrieben ein Schaum bereitet. 12,4 g Favor SXM 100 werden wie beschrieben in den Schaum eingearbeitet und getrocknet.

Im Falle **(a)** wird ein stabiles, etwas flexibles Flächengebilde erhalten, im Falle **(b)** ist die entstandene Fläche instabil, sie läßt sich nicht mehr handhaben. Aufnahmecharakteristik: TBT: max./ret. [l/m2]/[l/m2] = 15,4/9,6 AUL (2∗103Pa) = 9,5 l/m2

### Beispiel 10

Beispiel 9 wird wiederholt. Jedoch wird statt der Cellulosefasern Kreide eingearbeitet. Das erhaltene Flächengebilde spröde, die Aufnahmewerte entsprechen den in Beispiel 9 gemessenen.

### Beispiel 11

Es wird wie in Beispiel 1 verfahren, jedoch wird zum Aufstreuen des Favors eine Schablone (vgl. Fig. 3) verwendet. Eine solche Schablone erlaubt eine für den Hygienebereich bevorzugte Anordnung des Superabsorbers in der flächenförmigen Matrix.
Das Absorptionsvermögen der so erhaltenen Fläche entpricht dem des eingesetzten Superabsorbers.

### Beispiel 12

Beispiel 11 wird wiederholt, jedoch wird nur die Hälfte des Wassers verwendet. Die so erhaltene Fläche ist härter und spröder und weniger voluminös als die nach Bsp. 11 erhaltene. Sie zeigt ein Absorptionsvermögen das dem des eingesetzten Superabsorbers entspricht.

### Beispiel 13

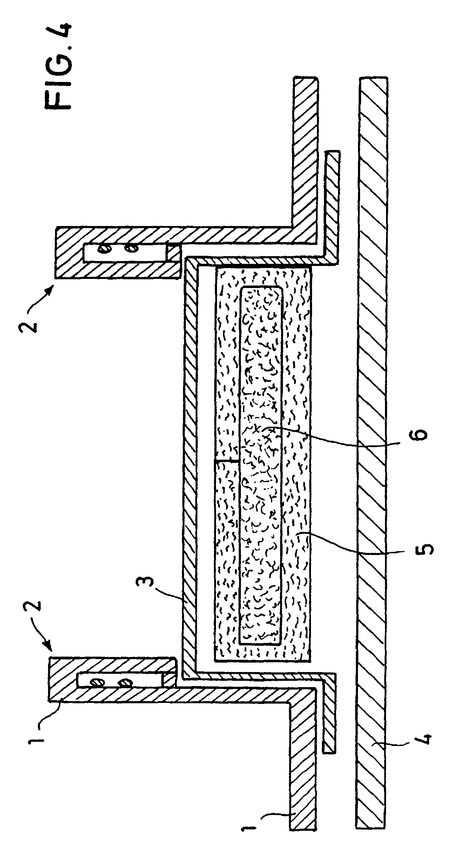

Aus der in Beispiel 5 hergestellten Flächengebilde wird gemäß Fig. 4 eine Windel konstruiert.
Die verwendete PE Folie und das Polypropylenabdeckflies wurden in einer für die Windelherstellung üblichen Qualität verwendet.
Die in Beispiel 5 hergestellte Fläche wird als Kern eingesetzt.

### Beispiel 14

10∗15 cm der in Beispiel 2 beschriebenen Fläche werden in eine Verpackungsschale gelegt und mit einem handelsüblichen Küchentuch (Kleenex) abgedeckt. Ein tiefgekühltes Hähnchen (850g) wird auf das Tuch gelegt.
Das gesamte Tauwasser (Testdauer 18 h) wird von der erfindungsgemäßen Fläche aufgesaugt.

### Beispiel 15

Beispiel 12 wird ohne Schablone wiederholt, auch wurde statt des Favors® Stockosorb ® 400 (schwachvernetztes Copolymer auf Basis von Acrylamid) verwendet. Aus dieser Fläche wurden Streifen mit der Größe 1∗7.5 cm ausgeschnitten. Acht der Streifen werden gänzlich in einen Erde enthaltenden zylindrischen Blumentopf (10 cm Höhe, Durchmesser 8,5 cm) gesteckt. Die Erde wurde 5 Tage feucht gehalten. Die Folie hatte sich dannach aufgelöst, das SAP befand sich in einer für z. B. die Pflanzenaufzucht geeigneten Anordnung in der Erde.

### Beispiel 16

Beispiel 12 wird ohne Schablone wiederholt, auch wird statt des Favors die gleiche Menge der in PCT/EP93/01060 Beispiel 9 genannten superabsorbierenden Depotmittelzubereitung verwendet.
1 cm² der so erhaltenen Fläche werden in einen Teebeutel eingeschweißt. Der Teebeutel wird eine Stunde in 50 ml einer 0,2 % igen Kochsalzlösung eingehängt.
Nach einer Stund wird die Kochsalzlösung erneuert.
Auch nach dem 5ten Zyklus zeigt die Blaufärbung der Kochsalzlösung eine Freisetzung des Wirkstoffs an.

## Patentansprüche

1. Schichtförmiges Absorptionsmittel für Wasser oder wäßrige Lösungen, bestehend aus wenigstens 2 Komponenten A und B, wobei Komponente A wenigstens ein wasserquellbares, synthetisches und/oder natürliches Polymeres ist und Komponente B wenigstens ein wasserlösliches, aufgeschäumtes, synthetisches und/oder natürliches Polymeres ist, **dadurch gekennzeichnet, daß** Komponente A in der als flächenförmig ausgebildeten Matrixkomponente B definiert ein- oder angebunden ist.

2. Schichtförmiges Absorptionsmittel nach Anspruch 1, **dadurch gekennzeichnet, daß** die Komponente A ein Polymer oder Copolymeres auf Basis von (Meth-)acrylsäure, (Meth)acrylnitril, (Meth-)acrylamid, Vinylacetat, Vinylalkohol, Vinylpyrrolidon, Vinylpyridin, Maleinsäure(anhydrid), Itakonsäure(anhydrid), Fumarsäure, Vinylsulfonsäure sowie die Amide, die N-Alkylderivate, die N,N-Dialkylderivate und die Ester dieser polymerisierbaren Säuren ist.

3. Schichtförmiges Absorptionsmittel nach Anspruch 1, **dadurch gekennzeichnet, daß** die Komponente A ein schwach vernetztes, natürliches Polymer oder ein Polymer nativen Ursprungs ist wie Guarkernmehl, Carboxymethylcellulose, Xanthan, Alginate, Gummi Arabicum, Chitin, Chitosan, Agar Agar, Hydroxyethylcellulose, Hydroxypropylcellulose, Methylcellulose, Stärke und Stärkederivate bzw. eine Mischung davon.

4. Schichtförmiges Absorptionsmittel nach Anspruch 1, **dadurch gekennzeichnet, daß** Komponente A eine Mischung aus zwei oder mehreren der in den Ansprüchen 2 und 3 genannten Komponenten ist.

5. Schichtförmiges Absorptionsmittel nach Anspruch 1, **dadurch gekennzeichnet, daß** die Komponente B ein wasserlösliches Polymer oder Copolymeres auf Basis von (Meth-)acrylsäure, (Meth-)acrylnitril, (Meth-)acrylamid, Vinylacetat, Vinylalkohol, Vinylpyrrolidon, Vinylpyridin, Maleinsäure(anhydrid), Itakonsäure (-anhydrid), Fumarsäure, Vinylsulfonsäure sowie die Amide, die N-Alkylderivate, die N,N dialkylderivate und die Ester dieser polymerisierbaren Säuren ist.

6. Schichtförmiges Absorptionsmittel nach Anspruch 1, **dadurch gekennzeichnet, daß** die Komponente B ein lösliches, natürliches Polymer oder ein Polymer nativen Ursprungs ist wie Guarkernmehl, Carboxymethylcellulose, Xanthan, Alginate, Gummi Arabicum, Chitin, Chitosan, Agar Agar, Hydroxyethylcellulose, Hydroxypropypcellulose, Methylcellulose, Stärke und Stärkederivate bzw. eine Mischung davon.

7. Schichtförmiges Absorptionsmittel nach den Ansprüchen 1,5,6, **dadurch gekennzeichnet, daß** Komponente B eine Mischung aus den Komponenten nach den Ansprüchen 5 und 6 ist.

8. Schichtförmiges Absorptionsmittel nach den Ansprüchen 1,5,6,7 **dadurch gekennzeichnet, daß** Komponente B aufgeschäumt ist und ein Schaumlitergewicht zwischen 10 g/l und 1500 g/l, vorzugsweise zwischen 25 g/l und 850 g/l und besonders bevorzugt zwischen 50 g/l und 500 g/l besitzt.

9. Schichtförmiges Absorptionsmittel nach einem der vorhergehenden Ansprüche, **dadurch gekennzeichnet, daß** das Verhältnis von B : A = 1 : 1000 - 100 : 1 liegt, bevorzugt sind Verhältnisse von B : A = 1 : 100 - 10 : 1, und besonders bevorzugt B : A = 1:25-2:1.

10. Schichtförmiges Absorptionsmittel nach einem der Ansprüche 1 - 9, **dadurch gekennzeichnet, daß** die Komponenten A und B teilweise chemisch miteinander reagiert haben.

11. Schichtförmiges Absorptionsmittel nach einem der Ansprüche 1 - 10, **dadurch gekennzeichnet, daß** die Komponenten A und B rein physikalisch miteinander verknüpft sind.

12. Schichtförmiges Absorptionsmittel nach einem der vorhergehenden Ansprüche in Form eines Blattes, einer Folie oder eines Rollgutes, eines Laminats oder eines anderen Schichtproduktes.

13. Verfahren zur Herstellung eines absorbierenden Körpers nach einem der Ansprüche 1-12, **dadurch gekennzeichnet, daß**:
a) eine Lösung der Komponente B in Wasser oder einer wäßrigen Lösung hergestellt wird und mit den üblichen, technisch bekannten Methoden aufgeschäumt wird,
b) dieser Schaum auf eine Fläche aufgebracht wird,
c) diese Fläche mit Komponente A bestreut wird
d) die erhaltene Flächenstruktur getrocknet wird.

14. Verfahren nach Anspruch 13, in dem die Schritte b und c mehrmals, ggf. mit zwischengeschalteten Trocknungen, wiederholt werden.

15. Verfahren nach Anspruch 13 - 14, in dem die Fläche abschließend gemäß Anspruch 13 Schritte b und d behandelt wird.

16. Verfahren nach Anspruch 13 - 15 in denen das auf eine Fläche bringen des Schaums durch Streichen, Rakeln, Sprühen, Gießen oder Pflatschen geschieht.

17. Verfahren nach einem der Ansprüche 13 - 16 in dem Komponente A durch spezielle Streuverfahren aufgebracht wird.

18. Verfahren nach Anspruch 17 in dem zum Aufstreuen Schablonen verwendet werden, welche eine definierte Anordnung der Komponente A in der Komponente B ermöglichen.

19. Verwendung der Wasser und wäßrige Flüssigkeiten absorbierenden Körper nach den Ansprüchen 1 - 12, **dadurch gekennzeichnet, daß** sie in Hygieneartikeln im sanitären und medizinischen Bereich zur Aufnahme von Wasser oder Körperflüssigkeiten verwendet werden.

20. Verwendung der Wasser und wäßrige Flüssigkeiten absorbierenden Körper nach den Ansprüchen 1 - 12, **dadurch gekennzeichnet, daß** sie direkt oder als Komponente in natürliche und/oder künstliche Böden zur Pflanzenzucht oder zum Transport und zur Lagerung von Pflanzen oder Pflanzenteilen verwendet werden.

21. Verwendung der Wasser und wäßrige Flüssigkeiten absorbierenden Körper nach den Ansprüchen 1 - 12, **dadurch gekennzeichnet, daß** sie als wasserblockierendes Isolationsmaterial für Rohre und Leitungen, insbesondere für elektrische und lichtleitende Kabel verwendet werden.

22. Verwendung der Wasser und wäßrige Flüssigkeiten absorbierenden Körper nach den Ansprüchen 1 - 12, **dadurch gekennzeichnet, daß** sie als wasserblockierendes Isolationsmaterial für Baukonstruktionen, insbesondere für Außenmauern verwendet werden.

23. Verwendung der Wasser und wäßrige Flüssigkeiten absorbierenden Körper nach den Ansprüchen 1 - 12, **dadurch gekennzeichnet, daß** sie direkt oder als flüssigkeitsaufnehmende und/oder flüssigkeitsspeichernde Komponente in Verpackungsmaterialien verwendet werden.

24. Verwendung der Wasser und wäßrige Flüssigkeiten absorbierenden Körper nach den Ansprüchen 1 - 12, **dadurch gekennzeichnet, daß** sie als Teil in Bekleidungsstücken verwendet werden.

25. Verwendung der Wasser und wäßrige Flüssigkeiten absorbierenden Körper nach den Ansprüchen 1 - 12, **dadurch gekennzeichnet, daß** sie als Speicher zur kontrollierten Freisetzung eines Wirkstoffs verwendet werden.

26. Chemisch-technische Produkte enthaltend eine Zusammensetzung nach den Ansprüchen 1 - 12 oder hergestellt gemäß den Ansprüchen 13 - 18.

## Claims

1. Layered absorbent for water or aqueous solutions, consisting of at least two components A and B, component A being at least a water-swellable, synthetic and/or natural polymer and component B being at least a water-soluble, foamed, synthetic and/or natural polymer, **characterised in that** component A is integrated or attached in a defined manner in the matrix component B designed so as to be sheet-like.

2. Layered absorbent according to claim 1, **characterised in that** component A is a polymer or copolymer based on (meth)acrylic acid, (meth)acrylonitrile, (meth)acrylamide, vinyl acetate, vinyl alcohol, vinyl pyrrolidone, vinyl pyridine, maleic acid(anhydride), itaconic acid(anhydride), fumaric acid, vinyl sulphonic acid and the amides, the N-alkyl derivatives, the N,N-dialkyl derivatives and the esters of these polymerisable acids.

3. Layered absorbent according to claim 1, **characterised in that** component A is a weakly cross-linked, natural polymer or a polymer of natural origin, such as guar gum, carboxymethylcellulose, xanthan gum, alginates, gum arabic, chitin, chitosan, agar agar, hydroxyethylcellulose, hydroxypropylcellulose, methylcellulose, starch and starch derivatives or a mixture thereof.

4. Layered absorbent according to claim 1, **characterised in that** component A is a mixture of two or more of the components mentioned in claims 2 and 3.

5. Layered absorbent according to claim 1, **characterised in that** component B is a water-soluble polymer or copolymer based on (meth)acrylic acid, (meth)acrylonitrile, (meth)acrylamide, vinyl acetate, vinyl alcohol, vinyl pyrrolidone, vinyl pyridine, maleic acid(anhydride), itaconic acid(anhydride), fumaric acid, vinyl sulphonic acid and the amides, the N-alkyl derivatives, the N,N-dialkyl derivatives and the esters of these polymerisable acids.

6. Layered absorbent according to claim 1, **characterised in that** component B is a soluble, natural polymer or a polymer of natural origin, such as guar gum, carboxymethylcellulose, xanthan gum, alginates, gum arabic, chitin, chitosan, agar agar, hydroxyethylcellulose, hydroxypropylcellulose, methylcellulose, starch and starch derivatives or a mixture thereof.

7. Layered absorbent according to claims 1, 5, 6, **characterised in that** component B is a mixture of the components according to claims 5 and 6.

8. Layered absorbent according to claims 1, 5, 6, 7 **characterised in that** component B is foamed and has a foam litre weight between 10 g/l and 1,500 g/l, preferably between 25 g/l and 850 g/l and most preferably between 50 g/l and 500 g/l.

9. Layered absorbent according to any of the preceding claims, **characterised in that** the ratio of B : A = 1 : 1,000 to 100 : 1, ratios of B : A = 1 : 100 to 10 : 1 being preferred and B : A = 1 : 25 to 2 : 1 being particularly preferred.

10. Layered absorbent according to any of claims 1 to 9, **characterised in that** components A and B have partially chemically reacted with one another.

11. Layered absorbent according to any of claims 1 to 10, **characterised in that** components A and B are coupled purely physically to one another.

12. Layered absorbent according to any of the preceding claims in the form of a leaf, a film or a rolled product, a laminate or another layered product.

13. Method for producing an absorbent body according to any of claims 1 to 12, **characterised in that**:
a) a solution of component B is produced in water or an aqueous solution and is foamed using the conventional technically known methods,
b) this foam is applied to a face,
c) this face is sprinkled with component A,
d) the face structure obtained is dried.

14. Method according to claim 13 in which steps b and c are repeated several times optionally with interposed dryings.

15. Method according to claims 13 to 14, in which the face is finally treated in accordance with claim 13, steps b and d.

16. Method according to claims 13 to 15 in which the foam is applied to a face by painting, doctoring, spraying, pouring or slop padding.

17. Method according to any of claims 13 to 16, in which component A is applied by special spreading methods.

18. Method according to claim 17 in which templates are used for spreading allowing defined arrangement of component A in component B.

19. Use of the water- and aqueous fluid-absorbing bodies according to claims 1 to 12, **characterised in that** they are used in hygienic products in the sanitary and medical sector to absorb water or bodily fluids.

20. Use of the water- and aqueous fluid-absorbing bodies according to claims 1 to 12, **characterised in that** they are used directly or as components in natural and/or synthetic soils for the cultivation of plants, or for transporting and storing plants or plant parts.

21. Use of the water- and aqueous fluid-absorbing bodies according to claims 1 to 12, **characterised in that** they are used as water-blocking insulating material for pipes and lines, in particular for electrical and light-guiding cables.

22. Use of the water- and aqueous fluid-absorbing bodies according to claims 1 to 12, **characterised in that** they are used as water-blocking insulating material for building constructions, in particular for external walls.

23. Use of the water- and aqueous fluid-absorbing bodies according to claims 1 to 12, **characterised in that** they are used directly or as fluid-absorbing and/or fluid-storing components in packaging materials.

24. Use of the water- and aqueous fluid-absorbing bodies according to claims 1 to 12, **characterised in that** they are used as a component in clothing.

25. Use of the water- and aqueous fluid-absorbing bodies according to claims 1 to 12, **characterised in that** they are used as storage mediums for controlled release of an active ingredient.

26. Chemical-technical products containing a composition according to claims 1 to 12 or produced in accordance with claims 13 to 18.

## Revendications

1. Agent d'absorption stratifié d'eau ou de solutions aqueuses, comprenant au moins 2 composants A et B, dans lequel le composant A est un polymère synthétique et/ou naturel gonflable dans l'eau, et le composant B est au moins un polymère synthétique et/ou naturel expansé soluble dans l'eau, **caractérisé en ce que** le composant A est incorporé ou injecté de manière définie dans le composant matriciel B conçu en forme de feuilles.

2. Agent d'absorption stratifié selon la revendication 1, **caractérisé en ce que** le composant A est un polymère ou un copolymère à base d'acide (méth)acrylique, de (méth)acrylonitrile, de (méth)acrylamide, d'acétate de vinyle, d'alcool vinylique, de vinylpyrrolidone, de vinylpyridine, d'acide (anhydride) maléique, d'acide (anhydride) itaconique, d'acide fumarique, d'acide vinylsulfonique, ainsi que des amides, des dérivés N-alkyle, des dérivés N,N-dialkyle et des esters de ces acides polymérisables.

3. Agent d'absorption stratifié selon la revendication 1, **caractérisé en ce que** le composant A est un polymère naturel faiblement réticulé ou un polymère d'origine naturelle tel que la farine de guar, la carboxyméthylcellulose, le xanthane, les alginates, la gomme arabique, la chitine, le chitosan, l'agar agar, l'hydroxyéthylcellulose, l'hydroxypropylcellulose, la méthylcellulose, l'amidon et les dérivés de l'amidon ou un mélange de ceux-ci.

4. Agent d'absorption stratifié selon la revendication 1, **caractérisé en ce que** le composant A est un mélange de deux ou de plusieurs composants mentionnés dans les revendications 2 et 3.

5. Agent d'absorption stratifié selon la revendication 1, **caractérisé en ce que** le composant B est un polymère ou un copolymère à base d'acide (méth)acrylique, de (méth)acrylonitrile, de (méth)acrylamide, d'acétate de vinyle, d'alcool vinylique, de vinylpyrrolidone, de vinylpyridine, d'acide (anhydride) maléique, d'acide (anhydride) itaconique, d'acide fumarique, d'acide vinylsulfonique, ainsi que des amides, des dérivés N-alkyle, des dérivés N,N-dialkyle et des esters de ces acides polymérisables.

6. Agent d'absorption stratifié selon la revendication 1, **caractérisé en ce que** le composant B est un polymère naturel faiblement réticulé ou un polymère d'origine naturelle tel que la farine de guar, la carboxyméthylcellulose, le xanthane, les alginates, la gomme arabique, la chitine, le chitosan, l'agar agar, l'hydroxyéthylcellulose, l'hydroxypropylcellulose, la méthylcellulose, l'amidon et les dérivés de l'amidon ou un mélange de ces derniers.

7. Agent d'absorption stratifié selon les revendications 1, 5, 6, **caractérisé en ce que** le composant B est un mélange des composants selon les revendications 5 et 6.

8. Agent d'absorption stratifié selon les revendications 1, 5, 6, 7, **caractérisé en ce que** le composant B est expansé et présente un poids par litre de mousse compris entre 10 g/l et 1500 g/l, de préférence compris entre 25 g/l et 850 g/l, et plus préférablement entre 50 g/l et 500 g/l.

9. Agent d'absorption stratifié selon l'une quelconque des revendications précédentes, **caractérisé en ce que** le rapport de B / A est égal à 1/1000 - 100/1, des rapports de B/A = 1/100 - 10/1 sont préférés, et des rapports de B/A = 1/25 - 2/1 sont particulièrement préférés.

10. Agent d'absorption stratifié selon l'une quelconque des revendications 1 à 9, **caractérisé en ce que** les composants A et B ont partiellement réagi chimiquement l'un avec l'autre.

11. Agent d'absorption stratifié selon l'une quelconque des revendications 1 à 10, **caractérisé en ce que** les composants A et B sont liés l'un à l'autre de manière purement physique.

12. Agent d'absorption stratifié selon l'une quelconque des revendications précédentes sous forme de feuille, de film ou de matière en rouleau, de stratifié ou d'autre produit en couches.

13. Procédé de fabrication d'un agent d'absorption selon l'une quelconque des revendications 1 - 12, **caractérisé en ce :**
a) une solution du composant B est préparée dans de l'eau ou dans une solution aqueuse puis expansée selon les méthodes habituelles connues dans l'art,
b) cette mousse est appliquée sur une surface,
c) cette surface est parsemée du composant A,
d) la structure superficielle obtenue est séchée.

14. Procédé selon la revendication 13, dans lequel les étapes b et c sont répétées à plusieurs reprises, éventuellement avec des séchages intermédiaires.

15. Procédé selon la revendication 13 ou 14, dans lequel la surface est finalement traitée selon la revendication 13 étapes b et d.

16. Procédé selon les revendications 13 à 15, dans lequel la mousse est appliquée sur une surface par enduction, raclage, pulvérisation, coulée ou placage.

17. Procédé selon l'une quelconque des revendications 13 à 16, dans lequel le composant A est appliqué à l'aide de procédés de dispersion spéciaux.

18. Procédé selon la revendication 17, dans lequel des gabarits sont utilisés pour la dispersion, lesquels permettent une disposition définie du composant A dans le composant B.

19. Utilisation des corps absorbant l'eau ou les liquides aqueux selon les revendications 1 à 12, **caractérisée en ce qu'**ils sont utilisés dans des articles hygiéniques dans les domaines sanitaire et médical pour l'absorption d'eau ou de liquides corporels.

20. Utilisation des corps absorbant l'eau ou les liquides aqueux selon les revendications 1 à 12, **caractérisée en ce qu'**ils sont utilisés directement ou en tant que composant dans des sols naturels et/ou artificiels pour la culture de plantes ou pour le transport et le stockage de plantes ou de parties de plantes.

21. Utilisation des corps absorbant l'eau ou des liquides aqueux selon les revendications 1 à 12, **caractérisée en ce qu'**ils sont utilisés en tant que matériau isolant bloquant l'eau pour des tuyaux et des conduites, notamment pour des câbles électriques et d'éclairage.

22. Utilisation des corps absorbant l'eau ou les liquides aqueux selon les revendications 1 à 12, **caractérisée en ce qu'**ils sont utilisés en tant que matériau isolant bloquant l'eau pour la construction dans le bâtiment, notamment les murs extérieurs.

23. Utilisation des corps absorbant l'eau ou les liquides aqueux selon les revendications 1 à 12, **caractérisée en ce qu'**ils sont utilisés directement ou en tant que composants absorbant les liquides et/ou retenant les liquides dans des matériaux d'emballage.

24. Utilisation des corps absorbant de l'eau ou des liquides aqueux selon les revendications 1 à 12, **caractérisée en ce qu'**ils sont utilisés en tant que pièces de vêtements.

25. Utilisation des corps absorbant l'eau ou les liquides aqueux selon les revendications 1 à 12, **caractérisée en ce qu'**ils sont utilisés en tant que réservoir pour la libération contrôlée d'un principe actif.

26. Produits chimico-techniques contenant une composition selon les revendications 1 à 12 ou réalisée selon les revendications 13 à 18.
